# EUROPEAN PATENT SPECIFICATION

(11) **EP 2 363 160 B1**
(45) Date of publication and mention of the grant of the patent: **18.09.2013**
(21) Application number: 11168664.8
(22) Date of filing: 03.12.2009
(51) Int. Cl.: A61M 5/20, A61M 5/145

(54) **Inductively coupled injector faceplate**
Induktiv gekoppelte Injektorblende
Dalle d'injecteur couplée inductivement

(30) Priority: 05.12.2008 US 120100 P
(43) Date of publication of application: 07.09.2011
(62) Divisional of application: 09764966.9
(73) Proprietor: Mallinckrodt LLC, Hazelwood, MO 63042 (US)
(72) Inventor: Neer, Charles, Cincinnati, OH Ohio 45202 (US)
(74) Representative: Chettle, Adrian John

(56) References cited:
- DE-A1- 10 359 735
- US-A- 5 814 900
- US-A- 6 159 183
- US-A1- 2006 079 765

## Description

### FIELD OF THE INVENTION

The present invention generally relates to injection systems and, more particularly, to transferring power between injection system components.

### BACKGROUND

Various medical procedures require that one or more medical fluids be injected into the patient. Medical imaging procedures oftentimes involve the injection of a contrast media into the patient, possibly along with saline or other fluids. Other medical procedures involve injecting one or more fluids into a patient for therapeutic purposes. Power injectors may be used for these types of applications.

A power injector generally includes what is commonly referred to as a powerhead. One or more syringes may be mounted to the powerhead in various manners (e.g., detachably; rear-loading; front-loading; side-loading). Each syringe typically includes what may be characterized as a syringe plunger, piston, or the like. Each such syringe plunger is designed to interface with (e.g., contact and/or temporarily interconnect with) an appropriate syringe driver that is incorporated into the powerhead, such that operation of the syringe driver axially advances the associated syringe plunger inside and relative to a barrel of the syringe. One typical syringe driver is in the form of a ram that is mounted on a threaded lead or drive screw. Rotation of the drive screw in one rotational direction advances the associated ram in one axial direction, while rotation of the drive screw in the opposite rotational direction advances the associated ram in the opposite axial direction.

US-A-2006/0079765 discloses a power injector which uses hall effect sensors to distinguish different syringe faceplates.

DE-A-1 0359735 discloses a metering device for dosing a solution, and an energy and data cable linking an infusion pump and a needle module. The pre-characterizing portion of claim 1 appended thereto may be derived from this document.

### SUMMARY

According to the invention there is provided a power injector according to claim 1 or 2.

A number of feature refinements and additional features are applicable to the present invention. These feature refinements and additional features may be used individually or in any combination. As such, each of the following features that will be discussed may be, but are not required to be, used with any other feature or combination of features.

The power injector may include components that allow the transfer of power and/or data between the faceplate and the powerhead while the faceplate and powerhead remain free from a direct electrically-conductive connection such as a wiring harness or mating electrically conductive contacts.

Power may be transferred from the powerhead to the faceplate through the inductively coupled first and second coils. The power injector may include a primary ferromagnetic core fixedly interconnected to the powerhead and operatively disposed proximate to the first coil. In this regard, at least a portion of the primary ferromagnetic core may be disposed within the first coil. The power injector may include a secondary ferromagnetic core fixedly interconnected to the faceplate and operatively disposed proximate to the second coil. The primary ferromagnetic core and the secondary ferromagnetic core may be disposed such that, when the faceplate is attached to the powerhead, the primary ferromagnetic core and the secondary ferromagnetic core together form a ferromagnetic core of a transformer that includes the first coil and the second coil. In another embodiment, the primary ferromagnetic core may be disposed such that it is disposed within both the first coil and the second coil when the faceplate is attached to the powerhead.

The power injector may be operable to deliver at least 7 watts of power to the faceplate through the first and second coils. The power injector may include a power delivery control module operable to regulate power delivery through the first coil to the faceplate.

Data may be transferred between the powerhead and/or other components of the power injector and the faceplate. In an embodiment, data may be transferred through the inductively coupled first and second coils. In this regard, the powerhead may include a communications module operatively interconnected to the first coil, and the faceplate may include a communications module operatively interconnected to the second coil. In an embodiment, data may be transferred between the powerhead and/or other components of the power injector and the faceplate through a wireless link that does not utilize the inductively coupled coils. Such wireless links may use Bluetooth®, Wi-Fi, active radio frequency identification (RFID), and/or any other appropriate wireless communication technology.

The faceplate may include a syringe reader capable of reading information regarding a syringe installed proximate to the faceplate. The syringe reader may be in the form of an electromagnetic device capable of electromagnetically reading data from and/or writing data to an appropriate data tag. The syringe reader may, for example, include an RFID reader capable of reading an RFID tag on a syringe installed proximate to the faceplate. The information read from the syringe RFID tag may include, for example, lot number, expiration date and/or time, contents, concentration, and/or fill volume. The syringe reader may also be operable to write data to the syringe. For example, the syringe reader, in the form of an RFID reader/writer, may be operable to write to an RFID tag of the faceplate. The syringe reader may draw power through the inductive coupling between the first coil of the powerhead and the second coil of the faceplate.

The faceplate of the power injector may include a syringe heater. The syringe heater may be operable to heat fluid within a syringe installed onto the faceplate. The syringe heater may draw power through the inductive coupling between the first coil of the powerhead and the second coil of the faceplate.

A number of feature refinements and additional features are separately applicable to each of above-noted aspects of the present invention. These feature refinements and additional features may be used individually or in any combination. Any feature of any other various aspects of the present invention that is intended to be limited to a "singular" context or the like will be clearly set forth herein by terms such as "only," "single," "limited to," or the like. Merely introducing a feature in accordance with commonly accepted antecedent basis practice does not limit the corresponding feature to the singular (e.g., indicating that a power injector includes "a syringe" alone does not mean that the power injector includes only a single syringe). Moreover, any failure to use phrases such as "at least one" also does not limit the corresponding feature to the singular (e.g., indicating that a power injector includes "a syringe" alone does not mean that the power injector includes only a single syringe). Finally, use of the phrase "at least generally" or the like in relation to a particular feature encompasses the corresponding characteristic and insubstantial variations thereof (e.g., indicating that a syringe barrel is at least generally cylindrical encompasses the syringe barrel being cylindrical).

Any "logic" that may be utilized by any of the various aspects of the present invention may be implemented in any appropriate manner, including without limitation in any appropriate software, firmware, or hardware, using one or more platforms, using one or more processors, using memory of any appropriate type, using any single computer of any appropriate type or a multiple computers of any appropriate type and interconnected in any appropriate manner, or any combination thereof. This logic may be implemented at any single location or at multiple locations that are interconnected in any appropriate manner (e.g., via any type of network).

Any power injector that may be utilized to provide a fluid discharge may be of any appropriate size, shape, configuration, and/or type. Any such power injector may utilize one or more syringe plunger drivers of any appropriate size, shape, configuration, and/or type, where each such syringe plunger driver is capable of at least bi-directional movement (e.g., a movement in a first direction for discharging fluid; a movement in a second direction for accommodating a loading of fluid or so as to return to a position for a subsequent fluid discharge operation), and where each such syringe plunger driver may interact with its corresponding syringe plunger in any appropriate manner (e.g., by mechanical contact; by an appropriate coupling (mechanical or otherwise)) so as to be able to advance the syringe plunger in at least one direction (e.g., to discharge fluid). Each syringe plunger driver may utilize one or more drive sources of any appropriate size, shape, configuration, and/or type. Multiple drive source outputs may be combined in any appropriate manner to advance a single syringe plunger at a given time. One or more drive sources may be dedicated to a single syringe plunger driver, one or more drive sources may be associated with multiple syringe plunger drivers (e.g., incorporating a transmission of sorts to change the output from one syringe plunger to another syringe plunger), or a combination thereof. Representative drive source forms include a brushed or brushless electric motor, a hydraulic motor, a pneumatic motor, a piezoelectric motor, or a stepper motor.

Any such power injector may be used for any appropriate application where the delivery of one or more medical fluids is desired, including without limitation any appropriate medical application (e.g., computed tomography or CT imaging; magnetic resonance imaging or MRI; single photon emission computed tomography or SPECT imaging; positron emission tomography or PET imaging; X-ray imaging; angiographic imaging; optical imaging; ultrasound imaging). Any such power injector may be used in conjunction with any component or combination of components, such as an appropriate imaging system (e.g., a CT scanner). For instance, information could be conveyed between any such power injector and one or more other components (e.g., scan delay information, injection start signal, injection rate).

Any appropriate number of syringes may be utilized with any such power injector in any appropriate manner (e.g., detachably; front-loaded; rear-loaded; side-loaded), any appropriate medical fluid may be discharged from a given syringe of any such power injector (e.g., contrast media, a radiopharmaceutical, saline, and any combination thereof), and any appropriate fluid may be discharged from a multiple syringe power injector configuration in any appropriate manner (e.g., sequentially, simultaneously), or any combination thereof. In one embodiment, fluid discharged from a syringe by operation of the power injector is directed into a conduit (e.g., medical tubing set), where this conduit is fluidly interconnected with the syringe in any appropriate manner and directs fluid to a desired location (e.g., to a catheter that is inserted into a patient, for instance for injection). Multiple syringes may discharge into a common conduit (e.g., for provision to a single injection site), or one syringe may discharge into one conduit (e.g., for provision to one injection site), while another syringe may discharge into a different conduit (e.g., for provision to a different injection site). In one embodiment, each syringe includes a syringe barrel and a plunger that is disposed within and movable relative to the syringe barrel. This plunger may interface with the power injector's syringe plunger drive assembly such that the syringe plunger drive assembly is able to advance the plunger in at least one direction, and possibly in two different, opposite directions.

As used herein, the term "fluidly interconnected" describes a relationship between components or entities where fluid is operable to flow in a predetermined flow path between the components or entities. For example, "an injection device fluidly interconnected to a patient" describes a configuration where fluid can flow from the injection device through any interconnecting devices (e.g., tubing, connectors) and into the patient.

### BRIEF DESCRIPTION OF THE FIGURES

Figure 1 is a schematic of one embodiment of a power injector.
Figure 2A is a perspective view of one embodiment of a portable stand-mounted, dual-head power injector.
Figure 2B is an enlarged, partially exploded, perspective view of a powerhead used by the power injector of Figure 2A.
Figure 2C is a schematic of one embodiment of a syringe plunger drive assembly used by the power injector of Figure 2A.
Figure 3 is a perspective view of one embodiment of a powerhead and an inductively coupled injector faceplate.
Figure 4 is a block diagram of a powerhead and faceplate operatively interconnected through a primary and a secondary coil.

### DETAILED DESCRIPTION

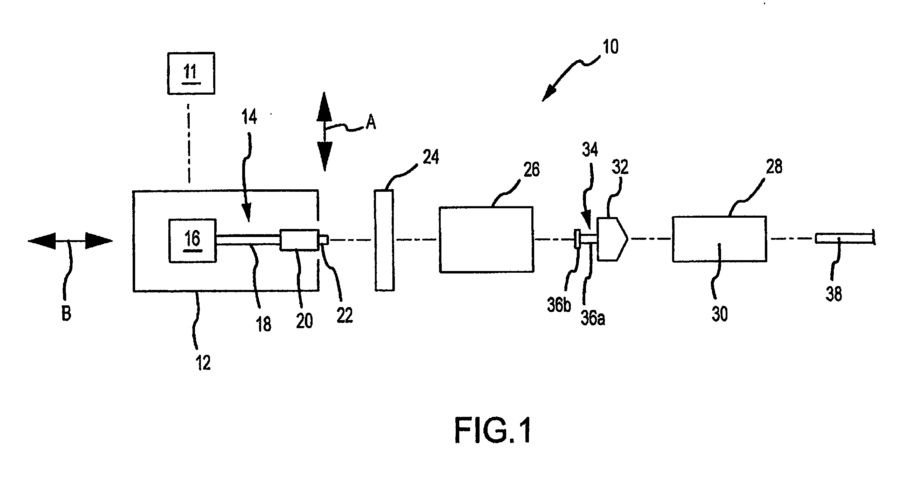

Figure 1 presents a schematic of one embodiment of a power injector 10 having a powerhead 12. One or more graphical user interfaces or GUIs 11 may be associated with the powerhead 12. Each GUI 11: 1) may be of any appropriate size, shape, configuration, and/or type; 2) may be operatively interconnected with the powerhead 12 in any appropriate manner; 3) may be disposed at any appropriate location; 4) may be configured to provide any of the following functions: controlling one or more aspects of the operation of the power injector 10; inputting/editing one or more parameters associated with the operation of the power injector 10; and displaying appropriate information (e.g., associated with the operation of the power injector 10); or 5) any combination of the foregoing. Any appropriate number of GUIs 11 may be utilized. In one embodiment, the power injector 10 includes a GUI 11 that is incorporated by a console that is separate from but which communicates with the powerhead 12. In another embodiment, the power injector 10 includes a GUI 11 that is part of the powerhead 12. In yet another embodiment, the power injector 10 utilizes one GUI 11 on a separate console that communicates with the powerhead 12, and also utilizes another GUI 11 that is on the powerhead 12. Each GUI 11 could provide the same functionality or set of functionalities, or the GUIs 11 may differ in at least some respect in relation to their respective functionalities.

A syringe 28 may be installed on the powerhead 12 and, when installed, may be considered to be part of the power injector 10. Some injection procedures may result in a relatively high pressure being generated within the syringe 28. In this regard, it may be desirable to dispose the syringe 28 within a pressure jacket 26. The pressure jacket 26 is typically associated with the powerhead 12 in a manner that allows the syringe 28 to be disposed therein as a part of or after installing the syringe 28 on the powerhead 12. The same pressure jacket 26 will typically remain associated with the powerhead 12, as various syringes 28 are positioned within and removed from the pressure jacket 26 for multiple injection procedures. The power injector 10 may eliminate the pressure jacket 26 if the power injector 10 is configured/utilized for low-pressure injections and/or if the syringe(s) 28 to be utilized with the power injector 10 is (are) of sufficient durability to withstand high-pressure injections without the additional support provided by a pressure jacket 26. In any case, fluid discharged from the syringe 28 may be directed into a conduit 38 of any appropriate size, shape, configuration, and/or type, which may be fluidly interconnected with the syringe 28 in any appropriate manner, and which may direct fluid to any appropriate location (e.g., to a patient).

The powerhead 12 includes a syringe plunger drive assembly or syringe plunger driver 14 that interacts (e.g., interfaces) with the syringe 28 (e.g., a plunger 32 thereof) to discharge fluid from the syringe 28. This syringe plunger drive assembly 14 includes a drive source 16 (e.g., a motor of any appropriate size, shape, configuration, and/or type, optional gearing, and the like) that powers a drive output 18 (e.g., a rotatable drive screw). A ram 20 may be advanced along an appropriate path (e.g., axial) by the drive output 18. The ram 20 may include a coupler 22 for interacting or interfacing with a corresponding portion of the syringe 28 in a manner that will be discussed below.

The syringe 28 includes a plunger or piston 32 that is movably disposed within a syringe barrel 30 (e.g., for axial reciprocation along an axis coinciding with the double-headed arrow B). The plunger 32 may include a coupler 34. This syringe plunger coupler 34 may interact or interface with the ram coupler 22 to allow the syringe plunger drive assembly 14 to retract the syringe plunger 32 within the syringe barrel 30. The syringe plunger coupler 34 may be in the form of a shaft 36a that extends from a body of the syringe plunger 32, together with a head or button 36b. However, the syringe plunger coupler 34 may be of any appropriate size, shape, configuration, and/or type.

Generally, the syringe plunger drive assembly 14 of the power injector 10 may interact with the syringe plunger 32 of the syringe 28 in any appropriate manner (e.g., by mechanical contact; by an appropriate coupling (mechanical or otherwise)) so as to be able to move or advance the syringe plunger 32 (relative to the syringe barrel 30) in at least one direction (e.g., to discharge fluid from the corresponding syringe 28). That is, although the syringe plunger drive assembly 14 may be capable of bi-directional motion (e.g., via operation of the same drive source 16), the power injector 10 may be configured such that the operation of the syringe plunger drive assembly 14 actually only moves each syringe plunger 32 being used by the power injector 10 in only one direction. However, the syringe plunger drive assembly 14 may be configured to interact with each syringe plunger 32 being used by the power injector 10 so as to be able to move each such syringe plunger 32 in each of two different directions (e.g. in different directions along a common axial path).

Retraction of the syringe plunger 32 may be utilized to accommodate a loading of fluid into the syringe barrel 30 for a subsequent injection or discharge, may be utilized to actually draw fluid into the syringe barrel 30 for a subsequent injection or discharge, or for any other appropriate purpose. Certain configurations may not require that the syringe plunger drive assembly 14 be able to retract the syringe plunger 32, in which case the ram coupler 22 and syringe plunger coupler 34 may not be desired. In this case, the syringe plunger drive assembly 14 may be retracted for purposes of executing another fluid delivery operation (e.g., after another pre-filled syringe 28 has been installed). Even when a ram coupler 22 and syringe plunger coupler 34 are utilized, these components may or may not be coupled when the ram 20 advances the syringe plunger 32 to discharge fluid from the syringe 28 (e.g., the ram 20 may simply "push on" the syringe plunger coupler 34 or directly on a proximal end of the syringe plunger 32). Any single motion or combination of motions in any appropriate dimension or combination of dimensions may be utilized to dispose the ram coupler 22 and syringe plunger coupler 34 in a coupled state or condition, to dispose the ram coupler 22 and syringe plunger coupler 34 in an un-coupled state or condition, or both.

The syringe 28 may be installed on the powerhead 12 in any appropriate manner. For instance, the syringe 28 could be configured to be installed directly on the powerhead 12. In the illustrated embodiment, a housing 24 is appropriately mounted on the powerhead 12 to provide an interface between the syringe 28 and the powerhead 12. This housing 24 may be in the form of an adapter to which one or more configurations of syringes 28 may be installed, and where at least one configuration for a syringe 28 could be installed directly on the powerhead 12 without using any such adapter. The housing 24 may also be in the form of a faceplate to which one or more configurations of syringes 28 may be installed. In this case, it may be such that a faceplate is required to install a syringe 28 on the powerhead 12 - the syringe 28 could not be installed on the powerhead 12 without the faceplate. When a pressure jacket 26 is being used, it may be installed on the powerhead 12 in the various manners discussed herein in relation to the syringe 28, and the syringe 28 will then thereafter be installed in the pressure jacket 26.

The housing 24 may be mounted on and remain in a fixed position relative to the powerhead 12 when installing a syringe 28. Another option is to movably interconnect the housing 24 and the powerhead 12 to accommodate installing a syringe 28. For instance, the housing 24 may move within a plane that contains the double-headed arrow A to provide one or more of coupled state or condition and an un-coupled state or condition between the ram coupler 22 and the syringe plunger coupler 34.

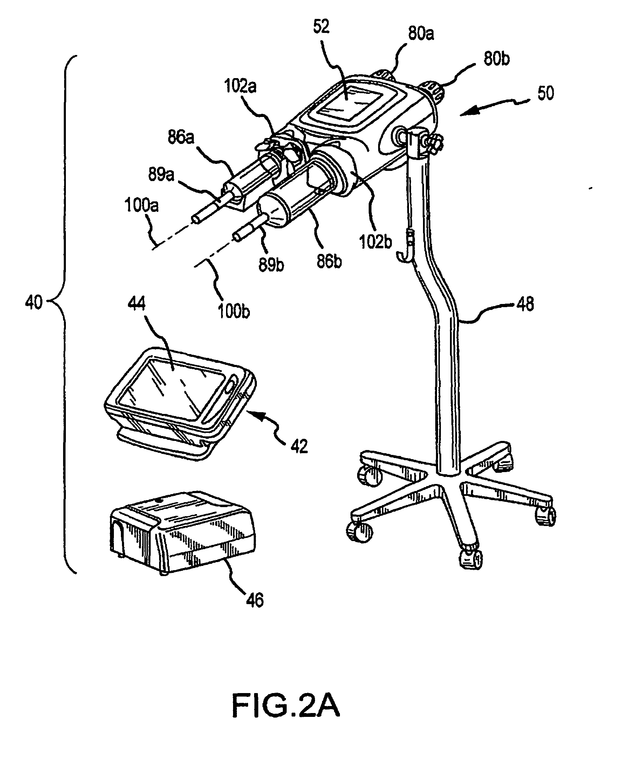

One particular power injector configuration is illustrated in Figure 2A, is identified by a reference numeral 40, and is at least generally in accordance with the power injector 10 of Figure 1. The power injector 40 includes a powerhead 50 that is mounted on a portable stand 48. A pair of syringes 86a, 86b for the power injector 40 are mounted on the powerhead 50. Fluid may be discharged from the syringes 86a, 86b during operation of the power injector 40.

The portable stand 48 may be of any appropriate size, shape, configuration, and/or type. Wheels, rollers, casters, or the like may be utilized to make the stand 48 portable. The powerhead 50 could be maintained in a fixed position relative to the portable stand 48. However, it may be desirable to allow the position of the powerhead 50 to be adjustable relative to the portable stand 48 in at least some manner. For instance, it may be desirable to have the powerhead 50 in one position relative to the portable stand 48 when loading fluid into one or more of the syringes 86a, 86b, and to have the powerhead 50 in a different position relative to the portable stand 48 for performance of an injection procedure. In this regard, the powerhead 50 may be movably interconnected with the portable stand 48 in any appropriate manner (e.g., such that the powerhead 50 may be pivoted through at least a certain range of motion, and thereafter maintained in the desired position).

It should be appreciated that the powerhead 50 could be supported in any appropriate manner for providing fluid. For instance, instead of being mounted on a portable structure, the powerhead 50 could be interconnected with a support assembly, that in turn is mounted to an appropriate structure (e.g., ceiling, wall, floor). Any support assembly for the powerhead 50 may be positionally adjustable in at least some respect (e.g., by having one or more support sections that may be repositioned relative to one or more other support sections), or may be maintained in a fixed position. Moreover, the powerhead 50 may be integrated with any such support assembly so as to either be maintained in a fixed position or so as to be adjustable relative the support assembly.

The powerhead 50 includes a graphical user interface or GUI 52. This GUI 52 may be configured to provide one or any combination of the following functions: controlling one or more aspects of the operation of the power injector 40; inputting/editing one or more parameters associated with the operation of the power injector 40; and displaying appropriate information (e.g., associated with the operation of the power injector 40). The power injector 40 may also include a console 42 and powerpack 46 that each may be in communication with the powerhead 50 in any appropriate manner (e.g., via one or more cables), that may be placed on a table or mounted on an electronics rack in an examination room or at any other appropriate location, or both. The powerpack 46 may include one or more of the following and in any appropriate combination: a power supply for the injector 40; interface circuitry for providing communication between the console 42 and powerhead 50; circuitry for permitting connection of the power injector 40 to remote units such as remote consoles, remote hand or foot control switches, or other original equipment manufacturer (OEM) remote control connections (e.g., to allow for the operation of power injector 40 to be synchronized with the x-ray exposure of an imaging system); and any other appropriate componentry. The console 42 may include a touch screen display 44, which in turn may provide one or more of the following functions and in any appropriate combination: allowing an operator to remotely control one or more aspects of the operation of the power injector 40; allowing an operator to enter/edit one or more parameters associated with the operation of the power injector 40; allowing an operator to specify and store programs for automated operation of the power injector 40 (which can later be automatically executed by the power injector 40 upon initiation by the operator); and displaying any appropriate information relation to the power injector 40 and including any aspect of its operation.

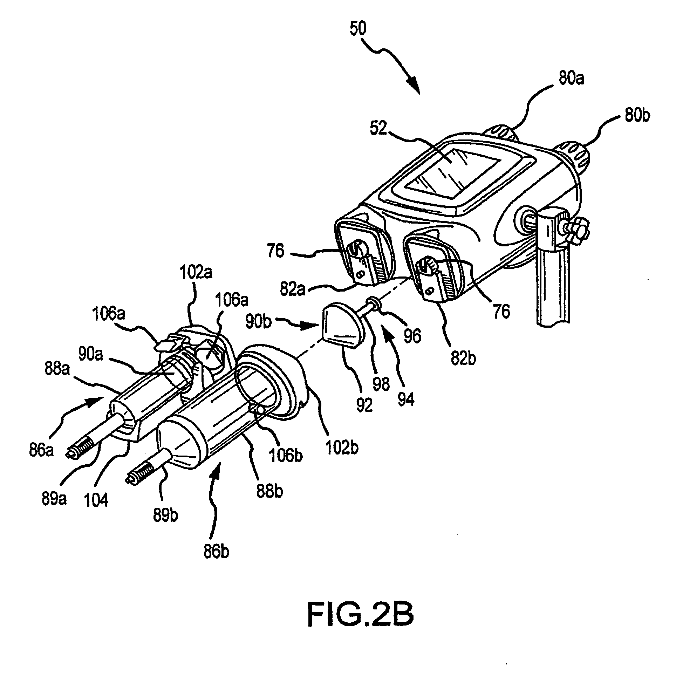

Various details regarding the integration of the syringes 86a, 86b with the powerhead 50 are presented in Figure 2B. Each of the syringes 86a, 86b includes the same general components. The syringe 86a includes plunger or piston 90a that is movably disposed within a syringe barrel 88a. Movement of the plunger 90a along an axis 100a (Figure 2A) via operation of the powerhead 50 will discharge fluid from within a syringe barrel 88a through a nozzle 89a of the syringe 86a. An appropriate conduit (not shown) will typically be fluidly interconnected with the nozzle 89a in any appropriate manner to direct fluid to a desired location (e.g., a patient). Similarly, the syringe 86b includes plunger or piston 90b that is movably disposed within a syringe barrel 88b. Movement of the plunger 90b along an axis 100b (Figure 2A) via operation of the powerhead 50 will discharge fluid from within the syringe barrel 88b through a nozzle 89b of the syringe 86b. An appropriate conduit (not shown) will typically be fluidly interconnected with the nozzle 89b in any appropriate manner to direct fluid to a desired location (e.g., a patient).

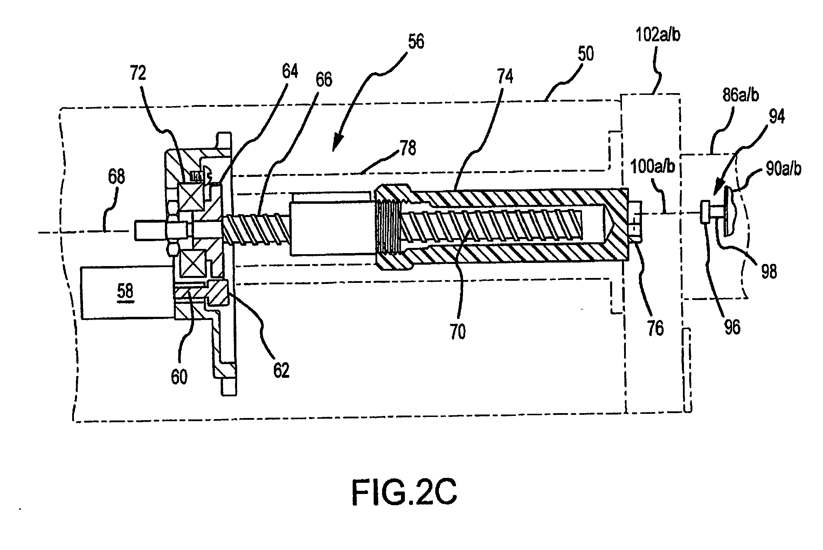

The syringe 86a is interconnected with the powerhead 50 via an intermediate faceplate 102a. This faceplate 102a includes a cradle 104 that supports at least part of the syringe barrel 88a, and which may provide/accommodate any additional functionality or combination of functionalities. A mounting 82a is disposed on and is fixed relative to the powerhead 50 for interfacing with the faceplate 102a. A ram coupler 76 of a ram 74 (Figure 2C), which are each part of a syringe plunger drive assembly or syringe plunger driver 56 (Figure 2C) for the syringe 86a, is positioned in proximity to the faceplate 102a when mounted on the powerhead 50. Details regarding the syringe plunger drive assembly 56 will be discussed in more detail below in relation to Figure 2C. Generally, the ram coupler 76 may be coupled with the syringe plunger 90a of the syringe 86a, and the ram coupler 76 and ram 74 (Figure 2C) may then be moved relative to the powerhead 50 to move the syringe plunger 90a along the axis 100a (Figure 2A). It may be such that the ram coupler 76 is engaged with, but not actually coupled to, the syringe plunger 90a when moving the syringe plunger 90a to discharge fluid through the nozzle 89a of the syringe 86a.

The faceplate 102a may be moved at least generally within a plane that is orthogonal to the axes 100a, 100b (associated with movement of the syringe plungers 90a, 90b, respectively, and illustrated in Figure 2A), both to mount the faceplate 102a on and remove the faceplate 102a from its mounting 82a on the powerhead 50. The faceplate 102a may be used to couple the syringe plunger 90a with its corresponding ram coupler 76 on the powerhead 50. In this regard, the faceplate 102a includes a pair of handles 106a. Generally and with the syringe 86a being initially positioned within the faceplate 102a, the handles 106a may be moved to in turn move/translate the syringe 86a at least generally within a plane that is orthogonal to the axes 100a, 100b (associated with movement of the syringe plungers 90a, 90b, respectively, and illustrated in Figure 2A). Moving the handles 106a to one position moves/translates the syringe 86a (relative to the faceplate 102a) in an at least generally downward direction to couple its syringe plunger 90a with its corresponding ram coupler 76. Moving the handles 106a to another position moves/translates the syringe 86a (relative to the faceplate 102a) in an at least generally upward direction to uncouple its syringe plunger 90a from its corresponding ram coupler 76.

The syringe 86b is interconnected with the powerhead 50 via an intermediate faceplate 102b. A mounting 82b is disposed on and is fixed relative to the powerhead 50 for interfacing with the faceplate 102b. A ram coupler 76 of a ram 74 (Figure 2C), which are each part of a syringe plunger drive assembly 56 for the syringe 86b, is positioned in proximity to the faceplate 102b when mounted to the powerhead 50. Details regarding the syringe plunger drive assembly 56 again will be discussed in more detail below in relation to Figure 2C. Generally, the ram coupler 76 may be coupled with the syringe plunger 90b of the syringe 86b, and the ram coupler 76 and ram 74 (Figure 2C) may be moved relative to the powerhead 50 to move the syringe plunger 90b along the axis 100b (Figure 2A). It may be such that the ram coupler 76 is engaged with, but not actually coupled to, the syringe plunger 90b when moving the syringe plunger 90b to discharge fluid through the nozzle 89b of the syringe 86b.

The faceplate 102b may be moved at least generally within a plane that is orthogonal to the axes 100a, 100b (associated with movement of the syringe plungers 90a, 90b, respectively, and illustrated in Figure 2A), both to mount the faceplate 102b on and remove the faceplate 102b from its mounting 82b on the powerhead 50. The faceplate 102b also may be used to couple the syringe plunger 90b with its corresponding ram coupler 76 on the powerhead 50. In this regard, the faceplate 102b may include a handle 106b. Generally and with the syringe 86b being initially positioned within the faceplate 102b, the syringe 86b may be rotated along its long axis 100b (Figure 2A) and relative to the faceplate 102b. This rotation may be realized by moving the handle 106b, by grasping and turning the syringe 86b, or both. In any case, this rotation moves/translates both the syringe 86b and the faceplate 102b at least generally within a plane that is orthogonal to the axes 100a, 100b (associated with movement of the syringe plungers 90a, 90b, respectively, and illustrated in Figure 2A). Rotating the syringe 86b in one direction moves/translates the syringe 86b and faceplate 102b in an at least generally downward direction to couple the syringe plunger 90b with its corresponding ram coupler 76. Rotating the syringe 86b in the opposite direction moves/translates the syringe 86b and faceplate 102b in an at least generally upward direction to uncouple its syringe plunger 90b from its corresponding ram coupler 76.

As illustrated in Figure 2B, the syringe plunger 90b includes a plunger body 92 and a syringe plunger coupler 94. This syringe plunger coupler 94 includes a shaft 98 that extends from the plunger body 92, along with a head 96 that is spaced from the plunger body 92. Each of the ram couplers 76 includes a larger slot that is positioned behind a smaller slot on the face of the ram coupler 76. The head 96 of the syringe plunger coupler 94 may be positioned within the larger slot of the ram coupler 76, and the shaft 98 of the syringe plunger coupler 94 may extend through the smaller slot on the face of the ram coupler 76 when the syringe plunger 90b and its corresponding ram coupler 76 are in a coupled state or condition. The syringe plunger 90a may include a similar syringe plunger coupler 94 for interfacing with its corresponding ram coupler 76.

The powerhead 50 is utilized to discharge fluid from the syringes 86a, 86b in the case of the power injector 40. That is, the powerhead 50 provides the motive force to discharge fluid from each of the syringes 86a, 86b. One embodiment of what may be characterized as a syringe plunger drive assembly or syringe plunger driver is illustrated in Figure 2C, is identified by reference numeral 56, and may be utilized by the powerhead 50 to discharge fluid from each of the syringes 86a, 86b. A separate syringe plunger drive assembly 56 may be incorporated into the powerhead 50 for each of the syringes 86a, 86b. In this regard and referring back to Figures 2A-B, the powerhead 50 may include hand-operated knobs 80a and 80b for use in separately controlling each of the syringe plunger drive assemblies 56.

Initially and in relation to the syringe plunger drive assembly 56 of Figure 2C, each of its individual components may be of any appropriate size, shape, configuration and/or type. The syringe plunger drive assembly 56 includes a motor 58, which has an output shaft 60. A drive gear 62 is mounted on and rotates with the output shaft 60 of the motor 58. The drive gear 62 is engaged or is at least engageable with a driven gear 64. This driven gear 64 is mounted on and rotates with a drive screw or shaft 66. The axis about which the drive screw 66 rotates is identified by reference numeral 68. One or more bearings 72 appropriately support the drive screw 66.

A carriage or ram 74 is movably mounted on the drive screw 66. Generally, rotation of the drive screw 66 in one direction axially advances the ram 74 along the drive screw 66 (and thereby along axis 68) in the direction of the corresponding syringe 86a/b, while rotation of the drive screw 66 in the opposite direction axially advances the ram 74 along the drive screw 66 (and thereby along axis 68) away from the corresponding syringe 86a/b. In this regard, the perimeter of at least part of the drive screw 66 includes helical threads 70 that interface with at least part of the ram 74. The ram 74 is also movably mounted within an appropriate bushing 78 that does not allow the ram 74 to rotate during a rotation of the drive screw 66. Therefore, the rotation of the drive screw 66 provides for an axial movement of the ram 74 in a direction determined by the rotational direction of the drive screw 66.

The ram 74 includes a coupler 76 that that may be detachably coupled with a syringe plunger coupler 94 of the syringe plunger 90a/b of the corresponding syringe 86a/b. When the ram coupler 76 and syringe plunger coupler 94 are appropriately coupled, the syringe plunger 90a/b moves along with ram 74. Figure 2C illustrates a configuration where the syringe 86a/b may be moved along its corresponding axis 100a/b without being coupled to the ram 74. When the syringe 86a/b is moved along its corresponding axis 100a/b such that the head 96 of its syringe plunger 90a/b is aligned with the ram coupler 76, but with the axes 68 still in the offset configuration of Figure 2C, the syringe 86a/b may be translated within a plane that is orthogonal to the axis 68 along which the ram 74 moves. This establishes a coupled engagement between the ram coupler 76 and the syringe plunger coupler 96 in the above-noted manner.

The power injectors 10, 40 of Figures 1 and 2A-C each may be used for any appropriate application, including without limitation for medical imaging applications where fluid is injected into a subject (e.g., a patient). Representative medical imaging applications for the power injectors 10, 40 include without limitation computed tomography or CT imaging, magnetic resonance imaging or MRI, single photon emission computed tomography or SPECT imaging, positron emission tomography or PET imaging, X-ray imaging, angiographic imaging, optical imaging, and ultrasound imaging. The power injectors 10, 40 each could be used alone or in combination with one or more other components. The power injectors 10, 40 each may be operatively interconnected with one or more components, for instance so that information may be conveyed between the power injector 10, 40 and one or more other components (e.g., scan delay information, injection start signal, injection rate).

Any number of syringes may be utilized by each of the power injectors 10, 40, including without limitation single-head configurations (for a single syringe) and dual-head configurations (for two syringes). In the case of a multiple syringe configuration, each power injector 10, 40 may discharge fluid from the various syringes in any appropriate manner and according to any timing sequence (e.g., sequential discharges from two or more syringes, simultaneous discharges from two or more syringes, or any combination thereof). Multiple syringes may discharge into a common conduit (e.g., for provision to a single injection site), or one syringe may discharge into one conduit (e.g., for provision to one injection site), while another syringe may discharge into a different conduit (e.g., for provision to a different injection site). Each such syringe utilized by each of the power injectors 10, 40 may include any appropriate fluid (e.g., a medical fluid), for instance contrast media, a radiopharmaceutical, saline, and any combination thereof. Each such syringe utilized by each of the power injectors 10, 40 may be installed in any appropriate manner (e.g., rear-loading configurations may be utilized; front-loading configurations may be utilized; side-loading configurations may be utilized).

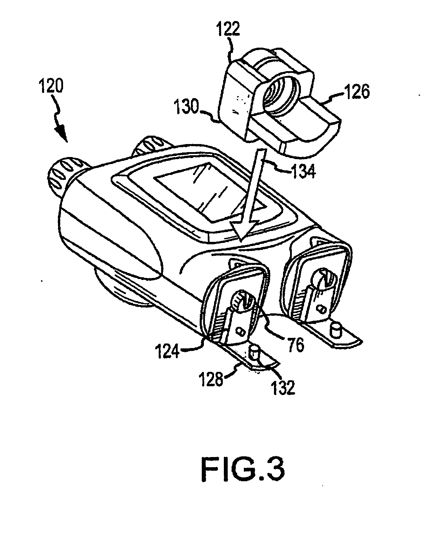

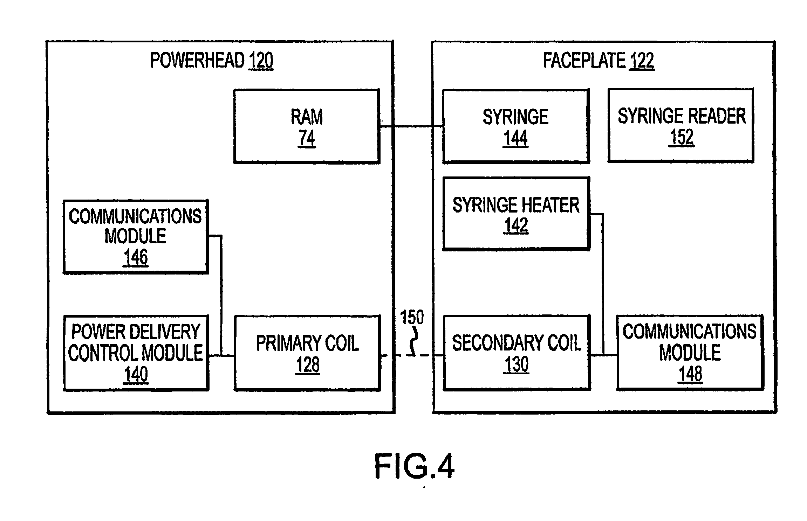

Figure 3 is a perspective view of one embodiment of a powerhead 120 and an inductively coupled injector faceplate 122. Figure 4 is a block diagram of the powerhead 120 and faceplate 122 operatively interconnected through a primary coil 128 and a secondary coil 130. Reference should be made to each of Figures 3 and 4 for the following discussion, as certain components may be shown in Figure 3 but not Figure 4, and vice versa. The term "faceplate" as used herein may refer to any removable syringe interface member disposed between and interconnected to the powerhead 120 and a syringe 144. In this regard, the faceplate 122 may be mountable to the powerhead 120 and the syringe 144 may be mountable to the faceplate 122. The powerhead 120 may be configured similarly to the powerhead 50 discussed above with reference to Figures 2A through 2C. For example, the powerhead 120 may include a faceplate mount 124 for mounting the faceplate 122 to the powerhead 120.

The powerhead 120 may also include componentry operable to inductively transfer power to the faceplate 122 when the faceplate 122 is mounted onto the powerhead 120. Data communication between the powerhead 120 and the faceplate 122 may be undertaken using at least some of the components that are also used for the inductive power transfer. In one embodiment, the powerhead 120 may be operable to inductively transfer up to 10 watts (W) or more of power from the powerhead 120 to the faceplate 122. Such power may be used, for example, to power a syringe heater 142 of the faceplate 122. The syringe heater 142 may be operable to heat fluid within the syringe 144 using a minimum power of about 7 W and a typical nominal power of about 10 W. To inductively transfer the power from the powerhead 120 to the faceplate 122, the powerhead 120 may include the primary coil 128 and the faceplate 122 may include the secondary coil 130. The power to the primary coil 128 may be controlled using any appropriate technique, such as pulse width modulation.

The primary coil 128 may be disposed such that a magnetic field generated by the primary coil 128, as electrical current is passed therethrough, intersects with the secondary coil 130 of the faceplate 122 when the faceplate 122 is attached to the powerhead 120. For example, and as illustrated in Figure 3, the primary coil 128 may be disposed such that it is at the bottom of the downward 134 sliding movement used to mount the faceplate 122 onto the powerhead 120 using the faceplate mount 124. Correspondingly, the secondary coil 130 of the faceplate 122 may be disposed toward the bottom of the faceplate 122.

A magnetic core 132 may be disposed such that a first portion of the magnetic core 132 is located within the interior of the primary coil 128. The second portion of the magnetic core 132 may be disposed such that it is located within the secondary coil 130 when the faceplate 122 is mounted to the powerhead 120. In this regard, the magnetic core 132 may serve to enhance the efficiency of the transfer of power from the primary coil 128 to the secondary coil 130. As illustrated in Figure 3, the entire magnetic core 132 may be fixedly interconnected to the powerhead 120.

In an alternate embodiment, the entirety of the magnetic core 132 may be fixedly interconnected to the faceplate 122. In such an embodiment, the second portion of the magnetic core 132 may be permanently disposed within the secondary coil 130 and the first portion of the magnetic core 132 may be disposed within the primary coil 128 upon installation of the faceplate 122 onto the powerhead 120.

In yet another alternate embodiment, the magnetic core 132 may be divided into two or more portions. In such an embodiment, a first portion of the magnetic core 132 may be permanently disposed within the primary coil 128 (as part of the powerhead 120) and a second portion of the magnetic core 132 may be permanently disposed within the secondary coil 130 (as part of the faceplate 122). In such an embodiment, upon installation of the faceplate 122 onto the powerhead 120, both the first and second portions of the magnetic core 132 may together act as a single magnetic core.

The magnetic core 132 may be shaped in any appropriate manner such as, for example, a straight cylinder rod, a U-shaped core, an E-shaped core, or a pot core. Furthermore, the magnetic core 132, the primary coil 128, and the secondary coil 130 may each be placed in any appropriate location such that an inductive coupling is formed when the faceplate 122 is mounted on the powerhead 120. Whether in a single unitary piece, or divided into two or more individual pieces, the magnetic core 132 may serve as the magnetic core for a transformer that includes the primary coil 128, the secondary coil 130, and the magnetic core 132. Such a transformer may be used, through inductive coupling, to transfer power between the powerhead 120 and the faceplate 122. Furthermore, and as discussed below, such an inductive coupling may also be used as a communications link between the powerhead 120 and the faceplate 122 (e.g., for data transfer).

The powerhead 120 may further include a power delivery control module 140 (Figure 4). The power delivery control module 140 may include electronic circuitry and/or software operable to control the amount of power delivered to the faceplate 122 through the primary coil 128. The power delivery control module 140 may be a stand-alone module or it may be incorporated into other portions of the powerhead 120. For example, the power delivery control module 140 may be incorporated into control circuitry and/or software of the powerhead 120. The power delivery control module 140 may be operable to deliver a variable level of power to the faceplate 122 dependent on current faceplate 122 power requirements. For example, the power requirements of the faceplate 122 may vary as a syringe heater 142 is cycled on and off. Other functions performed by electronic circuitry within the faceplate 122 may also require power. For example, the faceplate 122 may include a temperature sensor that may provide feedback to the powerhead 120 related to the current temperature of fluid within the syringe 144 mounted to the faceplate 122.

The primary coil 128 may be completely sealed within a casing of the powerhead 120. In this regard, the operation and performance of the primary coil 128 and the power delivery control module 140 may be unaffected by the presence of fluids. Such fluids may, for example, be due to spills associated with the fluids to be injected from the syringe 144 mounted to the faceplate 122. The magnetic core 132 may also be sealed within the housing of the powerhead 120. In this regard, typical problems (e.g., short-circuit, intermittent contact) associated with electrical connections (e.g. exposed contact points and electrical connectors) being exposed to fluids may be avoided.

The faceplate 122 may include a syringe mount 126. The syringe mount 126 may be configured to receive and hold the syringe 144. The syringe mount 126 may be configured to only hold a particular syringe or type of syringe. Alternatively, the syringe mount 126 may be configured to hold one or more different types of syringes.

Turning to Figure 4, the detachable faceplate 122 may be mounted to the powerhead 120. The detachable faceplate 122 may be configured similarly to the faceplates 102a/b discussed above. The powerhead 120 includes the ram 74 that may be connected to the syringe 144, that is in turn mounted to the faceplate 122. The ram 74 may be connected to the syringe 144 via, for example, the ram coupler 76 and head 96 shown in Figure 2B. The powerhead 120 further includes the primary coil 128 that may be inductively interconnected (represented by the dashed line 150) to the secondary coil 130 of the faceplate 122.

To facilitate communications between the powerhead 120 and the faceplate 122, the powerhead 120 may include a communications module 146. The faceplate 122 may include a corresponding communications module 148. The communications modules 146, 148 may include electronic circuitry and/or software. The communications modules 146, 148 may be stand-alone modules or they may be incorporated into other components. For example, the communications module 146 may be incorporated into control circuitry and/or software of the powerhead 120.

The communications module 146 may, as illustrated in Figure 4, be interconnected to the primary coil 128 and may be operative to communicate with communications module 148 of the faceplate 122 through the inductive coupling between the primary coil 128 and the secondary coil 130. Such communications may be performed in conjunction with the delivery of power to the faceplate 122 from the power delivery control module 140 through the primary coil 128 and the secondary coil 130. Power to operate the communications module 148 of the faceplate 122 may be delivered to the faceplate 122 through the inductive coupling between the primary coil 128 and the secondary coil 130. In this regard, the power injector 40 may be configured without any direct-contact electrical connection (e.g., cable, conductive pads, wires) between the powerhead 120 and the attached faceplate 122. Therefore, at least some components (e.g., primary coil 128 and secondary coil 130) may be used for both power transfer and data transfer.

In an alternate embodiment to that presented in Figure 4, the communications modules 146, 148 may each be wireless modules operable to communicate with each other without using (except for the deliver of power) the primary coil 128 and the secondary coil 130. For example, communications modules 146, 148 may be Bluetooth (TM) compatible and operable to communicate with each other using the Bluetooth (TM) protocol. The communications modules 146, 148 may use any other appropriate protocol or combination of protocols (e.g., custom protocols, Zigbee, active RFID, Wi-Fi) to communicate therebetween.

The wireless communication capability of the communications modules 146, 148, along with the wireless transmission of power discussed above, allows the powerhead 120 and faceplate 122 to be sealed. In this regard, communications between the powerhead 120 and the faceplate 122 may be unaffected by the presence of fluids or other contaminants disposed on the powerhead 120 and the faceplate 122.

As noted above, the faceplate 122 may include a syringe heater 142. The syringe heater 142 may receive power from the powerhead 120 through the secondary coil 130. The powerhead 120 and/or the faceplate 122 may include one or more temperature sensors and may be operable to determine the temperature of fluid disposed within the syringe 144. The fluid temperature may be used to control the syringe heater 142. The target temperature for the fluid within the syringe 144 may be user selectable.

The faceplate 122 may include any appropriate device for interfacing and/or communicating with the syringe 144. For example, the faceplate 122 may include a syringe reader 152. The syringe reader may be in the form of an electromagnetic device capable of electromagnetically reading data from and/or writing data to an appropriate data tag. The syringe reader 152 may, for example, be in the form of an RFID reader operable to read information from an RFID tag of the syringe 144 and communicate the information to the powerhead via the communications modules 146, 148. Such an RFID reader may be powered through the inductive coupling between the primary coil 128 and the secondary coil 130. The RFID tag may contain information regarding the syringe 144 and/or its contents. The syringe reader 152 may also be operable to write information to the syringe 144 (e.g., to an RFID tag of the syringe 144). In another example, the syringe reader 152 may include sensors operable to sense properties of an installed syringe 144 and/or fluids within an installed syringe 144. Signals based on outputs from the sensors may be communicated to the powerhead 120 and/or other components of the power injector 40.

Several exemplary configurations of faceplates 122 and associated syringe heater 142 control schemes will now be described. In a first configuration, the faceplate 122 includes the secondary coil 130 and the syringe heater 142, but lacks any communications capabilities. In such a faceplate 122, the syringe heater 142 may include a temperature sensor and circuitry such that when the faceplate 122 inductively receives power from the powerhead 120, the syringe heater 142 is powered and heats the syringe 144 to a pre-set temperature. In such a configuration, there may be no feedback to the powerhead 120 of the temperature of the syringe 144.

In a second exemplary configuration, the faceplate 122 may include the features of the first configuration, along with the ability to communicate the current temperature of the syringe 144 and/or fluid within the syringe 144 to the powerhead 120 and/or other components of the power injector 40. In such a configuration, the powerhead 120 may inductively supply power to the faceplate 122 and then wait until the syringe 144 is at an acceptable temperature before starting an injection or other sequence.

In a third exemplary configuration, the faceplate 122 may include the features of the first configuration, along with the ability to receive a temperature set point from the powerhead 120 and/or other components of the power injector 40. In this regard, the powerhead 120 and/or other components of the power injector 40 may be operable to control the temperature of fluid within the syringe 144. Such capabilities may accommodate differing desired temperatures for different fluids and/or patients. Optionally, the faceplate 122 may include the ability to communicate the current temperature of the syringe 144 and/or fluid within the syringe 144 to the powerhead 120 and/or other components of the power injector 40 as in the second exemplary configuration.

In a fourth exemplary configuration, the powerhead 120 may be operable to determine the temperature of the syringe 144 and/or fluids within the syringe 144 independently from power inductively supplied to the faceplate 122 via the primary coil 128. In such a configuration, the faceplate 122 may be configured such that any power inductively delivered to the faceplate 122 is directed to the syringe heater 142. Accordingly, the powerhead 120 may control the temperature of the syringe 144 by controlling the amount of power inductively delivered to the faceplate 122.

Where appropriate, any of the above-described exemplary syringe heater 142 control configurations may be combined with any other faceplate 122 functions described herein. Moreover, the other faceplate 122 functions described herein may be present on faceplates 122 that do not include a syringe heater 142.

The foregoing description has been presented for purposes of illustration and description. Furthermore, the description is not intended to limit the invention to the form disclosed herein. Consequently, variations and modifications in accordance with the claims are within the scope of the present invention. The embodiments described hereinabove are further intended to explain best modes known of practicing the disclosure and to enable others skilled in the art to utilize it in such, or other embodiments and with various modifications required by the particular application(s) or use(s) of the present disclosure.

## Claims

1. A power injector comprising:
a powerhead (120), wherein said powerhead comprises a first coil (128); and
a detachable faceplate (122) interconnected to said powerhead, wherein said faceplate comprises a second coil (130), wherein said faceplate comprises a syringe mount (126), wherein when said faceplate is attached to said powerhead, said first coil and said second coil are positioned relative to each other such that said first coil and said second coil are inductively coupled,
**characterized in that**
said faceplate may be moved within a plane that is orthogonal to the axis associated with movement of a syringe plunger, to mount the faceplate on and remove the faceplate from a faceplate mount (124) on the powerhead, and wherein
said power injector further comprises a primary ferromagnetic core fixedly interconnected to said powerhead and operatively disposed proximate to said first coil, and a secondary ferromagnetic core fixedly interconnected to said faceplate and operatively disposed proximate to said second coil, wherein when said faceplate is attached to said powerhead, said primary ferromagnetic core and said secondary ferromagnetic core are disposed such that together they form a ferromagnetic core of a transformer that comprises said first coil and said second coil.

2. A power injector comprising:
a powerhead (120), wherein said powerhead comprises a first coil (128); and
a detachable faceplate (122) interconnected to said powerhead, wherein said faceplate comprises a second coil (130), wherein said faceplate comprises a syringe mount (126), wherein when said faceplate is attached to said powerhead, said first coil and said second coil are positioned relative to each other such that said first coil and said second coil are inductively coupled,
**characterized in that**
said faceplate may be moved within a plane that is orthogonal to the axis associated with movement of a syringe plunger to mount the faceplate on and remove the faceplate from a faceplate mount (124) on the powerhead, and wherein
a magnetic core (132) is fixedly interconnected to said faceplate, wherein a second portion of the magnetic core is permanently disposed within the second coil, and a first portion of the magnetic core is disposed within the first coil when said faceplate is attached to the powerhead, so as to form a transformer that comprises said first coil, said second coil, and said magnetic core.

3. The power injector of any preceding claim, wherein said first coil is operable to transfer electrical power to said faceplate.

4. The power injector of claim 3, wherein said powerhead further comprises a power delivery control module (140) operable to regulate power delivery through said first coil to said faceplate.

5. The power injector of any preceding claim, wherein said powerhead is operable to deliver at least 7 watts of power to said faceplate through said first and second coils.

6. The power injector of any preceding claim, wherein said powerhead further comprises a wireless communications module (146) operable to broadcast and receive data wirelessly, wherein said wireless communications module is operable to broadcast and receive data wirelessly through said first coil.

7. The power injector of any one of claims 1 through 5, wherein said powerhead further comprises a wireless communications module operable to broadcast and receive data wirelessly.

8. The power injector of any preceding claim, wherein said faceplate comprises an RFID reader (152).

9. The power injector of any preceding claim, wherein said faceplate comprises a syringe heater (142).

10. The power injector of claims 1-7, wherein said faceplate comprises a read device operable to read data from a syringe attached to said faceplate.

11. The power injector of any preceding claim, wherein said faceplate comprises a read/write device operable to read data from and write data to a syringe installed on said syringe mount.

12. The power injector of any preceding claim, wherein said faceplate comprises a faceplate wireless communications module (148),
wherein said faceplate wireless communications module is operable to broadcast and receive data wirelessly through said second coil.

13. The power injector of any preceding claim, wherein said faceplate is operable to wirelessly communicate with said powerhead.

14. The power injector of any preceding claim, wherein said faceplate is free from a direct electrically conductive interconnection to said powerhead.

## Patentansprüche

1. Spritzenpumpe mit:
einem Triebkopf (120), wobei der Triebkopf (120) eine erste Spule (128) umfasst; und
einer abnehmbaren Blende (122), die mit dem Triebkopf verbunden ist, wobei die Blende eine zweite Spule (130) umfasst, wobei die Blende eine Spritzenaufnahme (126) umfasst, wobei die erste Spule und die zweite Spule relativ zueinander so angeordnet sind, dass die erste Spule und die zweite Spule induktiv gekoppelt sind, wenn die Blende an dem Triebkopf angebracht ist,
**dadurch gekennzeichnet, dass**
die Blende innerhalb einer Ebene bewegbar ist, die senkrecht zu der Achse ist, die zu der Bewegung eines Spritzenkolbens gehört, um die Blende an einer Blendenhalterung (124) an dem Triebkopf anzubringen und die Blende davon zu entfernen, und wobei
die Spritzenpumpe weiterhin einen primären ferromagnetischen Kern aufweist, der fest mit dem Triebkopf verbunden ist und operativ in der Nähe der ersten Spule angeordnet ist, und einen sekundären ferromagnetischen Kern, der fest mit der Blende verbunden und operativ in der Nähe der zweiten Spule angeordnet ist, wobei der primäre ferromagnetische Kern und der sekundäre ferromagnetische Kern so angeordnet sind, dass sie zusammen einen ferromagnetischen Kern eines Transformators bilden, der die erste Spule und die zweite Spule umfasst, wenn die Blende an dem Triebkopf angebracht ist.

2. Spritzenpumpe mit:
einem Triebkopf (120), wobei der Triebkopf eine erste Spule (128) umfasst; und
einer abnehmbaren Blende (122, die mit dem Triebkopf verbunden ist,
wobei die Blende eine zweite Spule (130) umfasst, wobei die Blende eine Spritzenaufnahme (126) umfasst, wobei die erste Spule und die zweite Spule relativ zueinander so angeordnet sind, dass die erste Spule und die zweite Spule induktiv gekoppelt sind, wenn die Blende an dem Triebkopf angebracht ist,
**dadurch gekennzeichnet, dass**
die Blende innerhalb einer Ebene bewegbar ist, die senkrecht zu der Achse ist, die zu der Bewegung eines Spritzenkolbens gehört, um die Blende an einer Blendenhalterung (124) an dem Triebkopf anzubringen und die Blende davon zu entfernen, und wobei
ein magnetischer Kern (132) fest mit der Blende verbunden ist, wobei ein zweiter Abschnitt des magnetischen Kerns permanent in der zweiten Spule angeordnet ist, und ein erster Abschnitt des magnetischen Kerns in der ersten Spule angeordnet ist, wenn die Blende an dem Triebkopf angebracht ist, so dass ein Transformator gebildet wird, der die erste Spule, die zweite Spule und den magnetischen Kern umfasst.

3. Spritzenpumpe nach einem der vorhergehenden Ansprüche, wobei die erste Spule betreibbar ist, um elektrische Leistung an die Blende zu übertragen.

4. Spritzenpumpe nach Anspruch 3, wobei der Triebkopf weiterhin ein Stromzufuhrsteuermodul (140) umfasst, das betreibbar ist, um die Stromzufuhr durch die erste Spule an die Blende zu regeln.

5. Spritzenpumpe nach einem der vorhergehenden Ansprüche, wobei der Triebkopf dazu fähig ist, mindestens sieben Watt Leistung über die ersten und zweiten Spulen an die Blende zu liefern.

6. Spritzenpumpe nach einem der vorhergehenden Ansprüche, wobei der Triebkopf weiterhin ein drahtloses Kommunikationsmodul (146) umfasst, das betreibbar ist, um Daten drahtlos zu senden und zu empfangen, wobei das drahtlose Kommunikationsmodul betreibbar ist, drahtlos Daten über die erste Spule zu senden und zu empfangen.

7. Spritzenpumpe nach einem der Ansprüche 1 bis 5, wobei der Triebkopf weiterhin ein drahtloses Kommunikationsmodul aufweist, das betreibbar ist, um Daten drahtlos zu senden und zu empfangen.

8. Spritzenpumpe nach einem der vorhergehenden Ansprüche, wobei die Blende einen RFID-Leser (152) umfasst.

9. Spritzenpumpe nach einem der vorhergehenden Ansprüche, wobei die Blende eine Spritzenheizung (142) umfasst.

10. Spritzenpumpe nach einem der Ansprüche 1 bis 7, wobei die Blende eine Lesevorrichtung umfasst, die betreibbar ist, um Daten von einer Spritze zu lesen, die an der Blende befestigt ist.

11. Spritzenpumpe nach einem der vorhergehenden Ansprüche, wobei die Blende eine Schreib-/Lesevorrichtung umfasst, die betreibbar ist, um Daten von einer an der Spritzenaufnahme angebrachten Spritze zu lesen und auf diese zu schreiben.

12. Spritzenpumpe nach einem der vorhergehenden Ansprüche, wobei die Blende ein drahtloses Blendenkommunikationsmodul (148) umfasst,
wobei das drahtlose Blendenkommunikationsmodul (148) betreibbar ist, Daten drahtlos durch die zweite Spule zu senden und zu empfangen.

13. Spritzenpumpe nach einem der vorhergehenden Ansprüche, wobei die Blende betreibbar ist, um drahtlos mit dem Triebkopf zu kommunizieren.

14. Spritzenpumpe nach einem der vorhergehenden Ansprüche, wobei die Blende frei von einer direkten elektrisch leitenden Verbindung mit dem Triebkopf ist.

## Revendications

1. Injecteur sous pression comprenant :
une tête d'alimentation (120), dans lequel ladite tête d'alimentation comprend une première bobine (128) ; et
une plaque frontale amovible (122) reliée à ladite tête d'alimentation, dans lequel ladite plaque frontale comprend une seconde bobine (130), dans lequel ladite plaque frontale comprend un support de seringue (126), dans lequel lorsque ladite plaque frontale est fixée à ladite tête d'alimentation, ladite première bobine et ladite seconde bobine sont positionnées l'une par rapport à l'autre de sorte que ladite première bobine et ladite seconde bobine sont couplées par induction,
**caractérisé en ce que**
ladite plaque frontale peut être déplacée dans un plan qui est orthogonal à l'axe associé à un mouvement d'un piston de seringue, pour monter la plaque frontale sur et retirer la plaque frontale d'un support de plaque frontale (124) sur la tête d'alimentation, et dans lequel
ledit injecteur de pression comprend en outre un noyau ferromagnétique principal relié fixement à ladite tête d'alimentation et disposé opérationnellement à proximité de ladite première bobine, et un noyau ferromagnétique secondaire relié fixement à ladite plaque frontale et disposé opérationnellement à proximité de ladite seconde bobine, dans lequel lorsque ladite plaque frontale est fixée à ladite tête d'alimentation, ledit noyau ferromagnétique principal et ledit noyau ferromagnétique secondaire sont disposés de façon à former ensemble un noyau ferromagnétique d'un transformateur qui comprend ladite première bobine et ladite seconde bobine.

2. Injecteur sous pression comprenant :
une tête d'alimentation (120), dans lequel ladite tête d'alimentation comprend une première bobine (128) ; et
une plaque frontale amovible (122) reliée à ladite tête d'alimentation, dans lequel ladite plaque frontale comprend une seconde bobine (130), dans lequel ladite plaque frontale comprend un support de seringue (126), dans lequel lorsque ladite plaque frontale est fixée à ladite tête d'alimentation, ladite première bobine et ladite seconde bobine sont positionnées l'une par rapport à l'autre de sorte que ladite première bobine et ladite seconde bobine sont couplées par induction,
**caractérisé en ce que**
ladite plaque frontale peut être déplacée dans un plan qui est orthogonal à l'axe associé à un mouvement d'un piston de seringue, pour monter la plaque frontale sur et retirer la plaque frontale d'un support de plaque frontale (124) sur la tête d'alimentation, et dans lequel
un noyau magnétique (132) est relié fixement à ladite tête d'alimentation, dans lequel une seconde portion du noyau magnétique est disposée en permanence au sein de la seconde bobine, et une première portion du noyau magnétique est disposée au sein de la première bobine lorsque ladite plaque frontale est fixée à la tête d'alimentation, de façon à former un transformateur qui comprend ladite première bobine, ladite seconde bobine et ledit noyau magnétique.

3. Injecteur sous pression selon l'une quelconque des revendications précédentes, dans lequel ladite première bobine est utilisable pour transférer la puissance électrique à ladite plaque frontale.

4. Injecteur sous pression selon la revendication 3, dans lequel ladite tête d'alimentation comprend en outre un module de commande de distribution de puissance (140) utilisable pour réguler la distribution de puissance par l'intermédiaire de ladite première bobine à ladite plaque frontale.

5. Injecteur sous pression selon l'une quelconque des revendications précédentes, dans lequel ladite tête d'alimentation est utilisable pour distribuer une puissance d'au moins 7 watts à ladite plaque frontale par l'intermédiaire desdites première et seconde bobines.

6. Injecteur sous pression selon l'une quelconque des revendications précédentes, dans lequel ladite tête d'alimentation comprend en outre un module de communications sans fil (146) utilisable pour radiodiffuser et recevoir des données en mode sans fil, dans lequel ledit module de communications sans fil est utilisable pour radiodiffuser et recevoir des données en mode sans fil par l'intermédiaire de ladite première bobine.

7. Injecteur sous pression selon l'une quelconque des revendications 1 à 5, dans lequel ladite tête d'alimentation comprend en outre un module de communications sans fil utilisable pour radiodiffuser et recevoir des données en mode sans fil.

8. Injecteur sous pression selon l'une quelconque des revendications précédentes, dans lequel ladite plaque frontale comprend un lecteur RFID (152).

9. Injecteur sous pression selon l'une quelconque des revendications précédentes, dans lequel ladite plaque frontale comprend un chauffe-seringue (142).

10. Injecteur sous pression selon les revendications 1 à 7, dans lequel ladite plaque frontale comprend un dispositif de lecture utilisable pour lire des données à partir d'une seringue fixée à ladite plaque frontale.

11. Injecteur sous pression selon l'une quelconque des revendications précédentes, dans lequel ladite plaque frontale comprend un dispositif de lecture/écriture utilisable pour lire des données à partir de et écrire des données sur une seringue installée sur ledit support de seringue.

12. Injecteur sous pression selon l'une quelconque des revendications précédentes, dans lequel ladite plaque frontale comprend un module de communications sans fil de plaque frontale (148),
dans lequel ledit module de communications sans fil de plaque frontale est utilisable pour radiodiffuser et recevoir des données en mode sans fil par l'intermédiaire de ladite seconde bobine.

13. Injecteur sous pression selon l'une quelconque des revendications précédentes, dans lequel ladite plaque frontale est utilisable pour communiquer en mode sans fil avec ladite tête d'alimentation.

14. Injecteur sous pression selon l'une quelconque des revendications précédentes, dans lequel ladite plaque frontale est dépourvue d'une interconnexion électriquement conductrice directe à ladite tête d'alimentation.
